Europäisches Patentamt

European Patent Office

Office européen des brevets

(19)

(11) Veröffentlichungsnummer: **0 292 800**

**A2**

(12) # EUROPÄISCHE PATENTANMELDUNG

(21) Anmeldenummer: 88107656.6

(22) Anmeldetag: 13.05.88

(51) Int. Cl.4: **C07K 5/02 , C07D 239/42 , C07D 241/26 , A61K 31/135 , A61K 37/00 , A61K 31/495 , A61K 31/505**

(30) Priorität: 26.05.87 DE 3717631

(43) Veröffentlichungstag der Anmeldung:
30.11.88 Patentblatt 88/48

(84) Benannte Vertragsstaaten:
**AT BE CH DE ES FR GB IT LI NL SE**

(71) Anmelder: **Merck Patent Gesellschaft mit beschränkter Haftung**
**Frankfurter Strasse 250**
**D-6100 Darmstadt(DE)**

(72) Erfinder: **Raddatz, Peter, Dr.**
**Grafenstrasse 37**
**D-6100 Darmstadt(DE)**
Erfinder: **Sombroek, Johannes, Dr.**
**Robert-Koch-Strasse 32**
**D-6100 Darmstadt 13(DE)**
Erfinder: **Gante, Joachim, Dr.**
**Stormstrasse 4**
**D-6100 Darmstadt 12(DE)**
Erfinder: **Schmitges, Claus J., Dr.**
**Karolinger Strasse 5**
**D-6114 Gross-Umstadt(DE)**
Erfinder: **Minck, Klaus Otto, Dr.**
**Büchestrasse 8**
**D-6105 Ober-Ramstadt(DE)**

(54) Aminosäurederivate.

(57) Neue Aminosäurederivate der Formel I
$$X-V-CHR^2-CHOH-Z-(CH_2)_a-CO-NR^3-CHR^4-CR^5-(CHR^6)_n-CO-E-Q-Y \quad I$$
worin
$X$, $V$, $Z$, $E$, $Q$, $Y$, $R^2$, $R^3$, $R^4$, $R^5$, $R^6$, a und n die in Patentansprüch 1 angegebene Bedeutung haben,
sowie ihre Salze hemmen die Aktivität des menschlichen Plasmarenins.

EP 0 292 800 A2

**Aminosäurederivate**

Die Erfindung betrifft neue Aminosäurederivate der Formel I

X-V-CHR²-CHOH-Z-(CH₂)ₐ-CO-NR³-CHR⁴-CR⁵-(CHR⁶)ₙ-CO-E-Q-Y    I

worin

X    H, R¹, R¹-O-CₘH₂ₘ-CO-, R¹-CₘH₂ₘ-O-CO-, R¹-CₘH₂ₘ-CO-, R¹-SO₂-, (R¹-CₘH₂ₘ)-L(R⁷-CₚH₂ₚ)-CᵣH₂ᵣ-CO-, R¹-(NHCH₂CH₂)ₘ-NH-CH₂CO-, 9-Fluorenyl-CₘH₂ₘ-O-CO-oder (R¹-CₘH₂ₘ-(T)ₓ-CO-CᵣH₂ᵣ)-L(R⁷-CₚH₂ₚ)-CᵧH₂ᵧ-CO-,

V    -CH₂-, -O- oder -NR⁸-,

Z    -CH₂-, -CH=CH-, -C≡C-, -CH₂-O-, -CH₂-NR⁹- oder -CH₂-S-,

E    0 bis 2 peptidartig miteinander verbundene Aminosäurereste ausgewählt aus der Gruppe bestehend aus Abu, Ala, Cal, His, Ile, Leu, Met, Nle, Phe, Trp, Tyr und Val,

Q und T    jeweils O oder NH,

Y    -CₜH₂ₜ-R¹⁰ oder -CwH₂w-(CR¹¹)ₛ-CₜH₂ₜ-R¹⁰,

R¹, R², R⁴, R⁷ und R¹⁰    jeweils H, A, Ar, Aralkyl, Het, Het-alkyl, unsubstituiertes oder ein- oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

R³, R⁶, R⁸ und R⁹    jeweils H oder A,

R⁵ und R¹¹    jeweils (H, OH), (H, NH₂) oder =O,

L    CH oder N,

a, m, p, r, t, w und y    jeweils 0, 1, 2, 3, 4, 5, 6, 7, 8, 9 oder 10,

n und s    jeweils 1 oder 2,

x    0 oder 1,

Ar    unsubstituiertes oder ein- oder mehrfach durch A, AO, Hal, CF₃, HO, H₂NCO, HANCO, A₂NCO, H₂NSO₂, HANSO₂, A₂NSO₂, Hydroxyalkyl mit 1-8 C-Atomen, H₂N und/oder Aminoalkyl mit 1-8 C-Atomen substituiertes Phenyl oder unsubstituiertes Naphthyl,

Het    einen gesättigten oder ungesättigten 5- oder 6-gliedrigen heterocyclischen Rest mit 1-4 N-, O- und/oder S-Atomen, der mit einem Benzolring kondensiert und/oder ein- oder mehrfach durch A, AO, Hal, CF₃, HO, O₂N, Carbonylsauerstoff, H₂N, HAN, A₂N, AcNH, AS, ASO, ASO₂, AOOC, CN, H₂NCO, HOOC, H₂NSO₂, ASO₂NH, Ar, Ar-alkenyl, Hydroxyalkyl und/oder Aminoalkyl mit jeweils 1-8 C-Atomen substituiert sein kann und/oder dessen N- und/oder S-Heteroatome auch oxydiert sein können,

Hal    F, Cl, Br oder J,

Ac    A-CO-, Ar-CO- oder A-NH-CO- und

A    Alkyl mit 1-8 C-Atomen bedeuten,

worin ferner an Stelle einer oder mehrerer -NH-COGruppen auch eine oder mehrere -NA-CO-Gruppen stehen können,
sowie deren Salze.

Ähnliche Verbindungen sind aus der EP-A-163237 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasmarenins. Diese Wirkung kann z. B. nach der Methode von F. Fyhrquist et. al., Clin.chem. 22, 250-256 (1976), nachgewiesen werden. Bemerkenswert ist, daß diese Verbindungen sehr spezifische Hemmer des Renins sind; für die Hemmung anderer Aspartylproteinasen (z. B. Pepsin und Kathepsin D) sind in der Regel wesentlich höhere (etwa 100 bis 10 000mal so hohe) Konzentrationen dieser Verbindungen notwendig.

Die Verbindungen können als Arzneimittelwirkstoffe in der Human- und Veterinärmedizin eingesetzt werden, insbesondere zur Prophylaxe und zur Behandlung von Herz-, Kreislauf- und Gefäßkrankheiten, vor allem von Hypertonie, Herzinsuffizienz und Hyperaldosteronismus. Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen. Solche diagnostische Tests können in der in der EP-A-77028 beschriebenen Weise durchgeführt werden.

Die vor- und nachstehend aufgeführten Abkürzungen von Aminosäureresten stehen für die Reste -NR-CR'R''-CO- (worin R, R' und R'' die für jede Aminosäure bekannte spezifische Bedeutung haben) folgender Aminosäuren:

Abu    2-Aminobuttersäure

Ala    Alanin

Cal    3-Cyclohexylalanin

Gly    Glycin

His    Histidin

Ile    Isoleucin

Leu    Leucin

Met    Methionin

Nle    Norleucin

Phe    Phenylalanin

Trp    Tryptophan

Tyr    Tyrosin

Val    Valin.

Ferner bedeutet nachstehend:

BOC    tert.-Butoxycarbonyl

BOM    Benzyloxymethyl

imi-BOM     Benzyloxymethyl in 1-Stellung des Imidazolrings

CBZ     Benzyloxycarbonyl

DNP     2,4-Dinitrophenyl

imi-DNP     2,4-Dinitrophenyl in 1-Stellung des Imidazolrings

FMOC     9-Fluorenylmethoxycarbonyl

IPOC     Isopropoxycarbonyl

MC     Morpholinocarbonyl

POA     Phenoxyacetyl

PyOC     2-(2-Pyridyl)-ethoxycarbonyl

DCCI     Dicyclohexylcarbodiimid

HOBt     1-Hydroxybenzotriazol.

Sofern die vorstehend genannten Aminosäuren in mehreren enantiomeren Formen auftreten können, so sind vor- und nachstehend, z. B. als Bestandteil der Verbindungen der Formel I, alle diese Formen und auch ihre Gemische (z. B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Gegenstand der Erfindung ist ferner ein Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt

oder daß man eine Carbonsäure der Formel II

X-V-CHR$^2$-CHOH-Z-(CH$_2$)$_a$-G$^1$-OH     II

worin G$^1$

(a) -CO-,

(b) -CO-W-,

(c) -CO-W-E$^1$-,

(d) -CO-W-E- und W -NR$^3$-CHR$^4$-CR$^5$-(CHR$^6$)$_n$-CO- bedeuten

oder eines ihrer reaktionsfähigen Derivate mit einer Verbindung der Formel III

H-G$^2$     III

worin G$^2$

(a) -W-E-Q-Y,

(b) -E-Q-Y,

(c) -E$^2$-Q-Y,

(d) -Q-Y und E$^1$ + E$^2$ zusammen E bedeuten

oder einem ihrer reaktionsfähigen Derivate umsetzt, oder daß man eine Verbindung der Formel IV

X-V-CHR$^2$-CO-Z-(CH$_2$)$_a$-CO-W-E-Q-Y     IV

mit einem Reduktionsmittel behandelt, und/oder daß man gegebenenfalls in einer Verbindung der Formel I eine funktionell abgewandelte Amino-und/oder Hydroxygruppe durch Behandeln mit solvolysierenden oder hydrogenolysierenden Mitteln in Freiheit setzt und/oder eine freie Amino- oder Hydroxygruppe acyliert und/oder eine Ketogruppe zu einer CHOH-Gruppe reduziert oder zu einer CH(NH$_2$)-Gruppe reduktiv aminiert und/oder eine Verbindung der Formel I durch Behandeln mit einer Säure in eines ihrer Salze überführt.

Vor- und nachstehend haben die Reste bzw. Parameter X, V, Z, E, Q, T, Y, R$^1$ bis R$^{11}$, L, a, m, n, p, r, s, t, w, x, y, Ar, Het, Hal, Ac, A, G$^1$, G$^2$, W, E$^1$ und E$^2$ die bei den Formeln I, II oder III angegebenen Bedeutungen, falls nicht ausdrücklich etwas anderes angegeben ist.

In den vorstehenden Formeln hat A 1 - 8, vorzugsweise 1,2,3 oder 4 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-l-methylpropyl, 1-Ethyl-2-methylpropyl, 1,1,2- oder 1,2,2-Trimethylpropyl, Heptyl, Octyl.

Cycloalkyl bedeutet vorzugsweise Cyclopropyl, Cyclobutyl, Cyclopentyl, Cyclohexyl oder Cycloheptyl, aber auch z. B. 1-, 2- oder 3-Methylcyclopentyl, 1-, 2-, 3- oder 4-Methylcyclohexyl.

Dementsprechend bedeutet Cycloalkyl-alkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohexylethyl, aber auch z. B. 1-, 2- oder 3-Methylcyclopentylmethyl, 1-, 2-, 3- oder 4-Methylcyclohexylmethyl.

Bicycloalkyl bedeutet vorzugsweise 1- oder 2-Dekalyl, 2-Bicyclo[2,2,1]heptyl oder 6,6-Dimethyl-2-bicyclo[3,1,1]heptyl.

Tricycloalkyl bedeutet vorzugsweise 1-Adamantyl.

Hal bedeutet vorzugsweise F, Cl oder Br, aber auch J.

Ac bedeutet vorzugsweise A-CO- wie Acetyl, Propionyl oder Butyryl, Ar-CO- wie Benzoyl, o-, m- oder p-Methoxybenzoyl oder 3,4-Dimethoxybenzoyl, A-NH-CO- wie N-Methyl- oder N-Ethylcarbamoyl.

Ar bedeutet vorzugsweise Phenyl, ferner bevorzugt o-, m- oder p-Tolyl, o-, m- oder p-Ethylphenyl, o-, m- oder p-Methoxyphenyl, o-, m- oder p-Fluorphenyl, o-, m- oder p-Chlorphenyl, o-, m- oder p-Bromphenyl, o-, m- oder p-Jodphenyl, o-, m- oder p-Trifluormethylphenyl, o-, moder p-Hydroxyphenyl, o-, m- oder p-Carbamoylphenyl, o-, m- oder p-(Methylcarbamomyl)-phenyl, o-, m- oder p-(Dimethylcarbamoyl)-phenyl, o-, m- oder p-Aminosulfonylphenyl, o-, m- oder p-(Methylaminosulfonyl)-phenyl, o-, m- oder p-(Dimethylaminosulfonyl)-phenyl, o-, m- oder p-Hydroxymethylphenyl, 2,3-, 2,4-, 2,5-, 2,6-, 3,4- oder 3,5-Dimethoxyphenyl, 3,4,5-Trimethoxyphenyl, o-, m- oder p-Aminophenyl, o-, m-oder p-Aminomethylphenyl, 1- oder 2-Naphthyl.

Dementsprechend bedeutet Ar-alkyl vorzugsweise Benzyl, 1- oder 2-Phenylethyl, o-, m- oder p-Methylbenzyl, 1- oder 2-o-, -m- oder -p-Tolylethyl, o-, m- oder p-Ethylbenzyl, 1- oder 2-o-, -m- oder -p-Ethylphenylethyl, o-, m- oder p-Methoxybenzyl, 1- oder 2-o-, -m- oder -p-Methoxyphenylethyl, o-, m- oder p-Fluorbenzyl, 1- oder 2-o-, -m- oder -p-Fluorphenylethyl, o-, m- oder p-Chlorbenzyl, 1- oder 2-o-, -m- oder -p-Chlorphenylethyl, o-, m- oder p-Brombenzyl, 1- oder 2-o-, -m- oder -p-Bromphenylethyl, o-, m- oder p-Jodbenzyl, 1- oder 2-o-, -m- oder -p-Jodphenylethyl, o-, m- oder p-Trifluormethylbenzyl, o-, m- oder p-Hydroxybenzyl, o-, m- oder p-Carbamoylbenzyl, o-, m- oder p-(Methylcarbamoyl)-benzyl, o-, m- oder p-(Dimethylcarbamoyl)-benzyl, o-, m- oder p-Aminosulfonylbenzyl, o-, m- oder p-(Methylaminosulfonyl)-benzyl, o-, m- oder p-(Dimethylaminosulfonyl)-benzyl, o-, m- oder p-Hydroxymethylbenzyl, 2,3-, 2,4-, 2,5-, 2,6-, 3,4- oder 3,5-Dimethoxybenzyl, 3,4,5-Trimethoxybenzyl, o-, m- oder p-Aminobenzyl, o-, m- oder p-Aminomethylbenzyl, 1- oder 2-Naphthylmethyl.

Het ist vorzugsweise 2- oder 3-Furyl, 2- oder 3-Thienyl, 1-, 2- oder 3-Pyrryl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidyl, weiterhin bevorzugt 1,2,3-Triazol-1-, -4- oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 2,1,5-Thiadiazol-3- oder -4-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6 oder 7Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7-, oder 8-Chinolyl, 1-, 3-, 4-, 5-, 6-, 7- oder 8-Isochinolyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Chinnolyl, 2-, 4-, 5-, 6-, 7- oder 8-Chinazolyl. Die heterocyclischen Reste können auch teilweise oder vollständig hydriert sein. Het kann also z. B. auch bedeuten 2,3-Dihydro-2-, -3-, -4- oder -5-furyl, 2,5-Dihydro-2-, -3-, -4- oder -5-furyl, Tetrahydro-2- oder -3-furyl, Tetrahydro-2- oder -3-thienyl, 2,3-Dihydro-1-, -2-, -3-, -4- oder -5-pyrryl, 2,5-Dihydro-1-, -2-, -3-, -4- oder -5-pyrryl, 1-, 2- oder 3-Pyrrolidinyl, Tetrahydro-1-, -2- oder -4-imidazolyl, 2,3-Dihydro-1-, -2-, -3-, -4- oder 5-pyrazolyl, 2,5-Dihydro-1-, -2-, -3-, -4- oder 5-pyrazolyl, Tetrahydro-1-, -3-, oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1,2,3,6-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidyl, 1-, 2- oder 3-piperazinyl, 1,2,3,4-Tetrahydro-1-, -2-, -3-, -4-, -5-, -6-, -7-, oder -8-chinolyl, 1,2,3,4-Tetrahydro-1-, -2-, -3-, -4-, -5-, -6-, -7- oder 8-isochinolyl.

Die heterocyclischen Reste können auch wie angegeben substituiert sein. Het kann also bevorzugt auch bedeuten: 2-Amino-4-thiazolyl, 4-Carboxy-2-thiazolyl, 4-Carbamoyl-2-thiazolyl, 4-(2-Aminoethyl)-2-thiazolyl, 2-Amino-5,6-dimethyl-3-pyrazinyl, 4-Carbamoylpiperidino, ferner z. B. 3-, 4- oder 5-Methyl-2-furyl, 2-, 4- oder 5-Methyl-3-furyl, 2,4-Dimethyl-3-furyl, 5-Nitro-2-furyl, 5-Styryl-2-furyl, 3-, 4- oder 5-Methyl-2-thienyl, 2-, 4- oder 5-Methyl-3-thienyl, 3-Methyl-5-tert.-butyl-2-thienyl, 5-Chlor-2-thienyl, 5-Phenyl-2- oder -3-thienyl, 1-, 3-, 4- oder 5-Methyl-2-pyrryl, 1-Methyl-4- oder -5-nitro-2-pyrryl, 3,5-Dimethyl-4-ethyl-2-pyrryl, 4-Methyl-5-pyrazolyl, 4- oder 5-Methyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 3-, 4-, 5- oder 6-Chlor-2-pyridyl, 2-, 4-, 5- oder 6-Chlor-3-pyridyl, 2- oder 3-Chlor-4-pyridyl, 2,6-Dichlorpyridyl, 2-Hydroxy-3-, -4-, -5- oder -6-pyridyl (= 1H-2-Pyridon-3-, -4-, -5- oder -6-yl), 5-Phenyl-1H-2-pyridon-3-yl, 5-p-Methoxyphenyl-1H-2-pyridon-3-yl, 2-Methyl-3-hydroxy-4-hydroxymethyl-5-pyridyl, 2-Hydroxy-4-amino-6-methyl-3-pyridyl, 3-N'-Methylureido-1H-4-pyridon-5-yl, 5- oder 6-Methyl-4-pyrimidyl, 2,6-Dihydroxy-4-pyrimidyl, 5-Chlor-2-methyl-4-pyrimidyl, 2-Methyl-4-amino-5-pyrimidyl, 3-Methyl-2-benzofuryl, 2-Ethyl-3-benzofuryl, 7-Methyl-2-benzothienyl, 1-, 2-, 4-, 5-, 6- oder 7-Methyl-3-indolyl, 1-Methyl-5- oder -6-benzimidazolyl, 1-Ethyl-5- oder -6-benzimidazolyl, 3-, 4-, 5-, 6-, 7- oder 8-Hydroxy-2-chinolyl.

$R^3$, $R^6$, $R^8$ und $R^9$ bedeuten vorzugsweise H oder Methyl, ferner Ethyl, Propyl, Isopropyl, Butyl oder Isobutyl. Falls n = 2 ist, können die beiden Reste $R^5$ gleich oder verschieden sein.

$R^2$ und $R^4$ bedeuten vorzugsweise Cyclohexylmethyl oder Benzyl, ferner bevorzugt A, insbesondere Methyl, Ethyl, Propyl, Isopropyl, Butyl, Isobutyl, sek.-Butyl, Pentyl, Isopentyl (3-Methylbutyl) oder 2-Methylbutyl, Phenyl, Benzyl, p-Chlorbenzyl, 1- oder 2-Naphthylmethyl, 2-Phenylethyl, 3-Phenylpropyl, 2-Cyclohexylethyl, 1- oder 2-Dekalylmethyl, Bicyclo[2,2,1] heptyl-2-methyl oder 6,6-Dimethylbicyclo[3,1,1]heptyl-2-methyl. Im einzelnen ist R² besonders bevorzugt Benzyl, R⁴ besonders bevorzugt Cyclohexylmethyl.

R⁵ ist vorzugsweise (H, OH).

R¹ und R⁷ bedeuten vorzugsweise A, insbesondere Methyl, Ethyl, Propyl, Isopropyl, Butyl, Isobutyl oder tert.-Butyl, weiterhin vorzugsweise Cyclopropyl, Cyclopentyl, Cyclohexyl, Methylcyclohexyl wie trans-4-Methylcyclohexyl, tert. Butylcyclohexyl wie trans-4-tert.-Butylcyclohexyl, Phenyl oder Benzyl.

R¹⁰ ist vorzugsweise H, A oder Het.

R¹¹ ist vorzugsweise = O.

Q ist vorzugsweise NH.

E fehlt vorzugsweise oder bedeutet vorzugsweise einen der angegebenen Aminosäurereste, insbesondere Ile.

Die Parameter a, m, p, r und x sind vorzugsweise 0, 1 oder 2; n und s sind vorzugsweise 1. Die Gruppen $C_tH_{2t}$ und $C_wH_{2w}$ sind vorzugsweise jeweils -CH₂-, -(CH₂)₂-, -CH(CH₃)-, -CH(Isobutyl)- oder -CH(sek.-Butyl).

X bedeutet vorzugsweise H; Alkyl wie Propyl oder Butyl; POA; Alkoxycarbonyl wie IPOC oder BOC; Ar-alkoxycarbonyl wie CBZ; Het-alkoxycarbonyl wie PyOC; Alkanoyl mit vorzugsweise 2-7 C-Atomen wie Acetyl, Propionyl, Butyryl, Isobutyryl, Hexanoyl, 3,3-Dimethylbutyryl, Heptanoyl, 2-Propylvaleryl ( = Dipropylacetyl); Cycloalkylcarbonyl wie Cyclopentylcarbonyl oder Cyclohexylcarbonyl; Ar-CO wie Benzoyl; Ar-alkanoyl wie Phenylacetyl, 2- oder 3-Phenylpropionyl, 2- oder 3-o-, -m- oder -p-Fluorphenylpropionyl, 2- oder 3-o-, -m- oder -p-Chlorphenylpropionyl, 4-Phenylbutyryl, 2-Benzyl-butyryl, 2-Benzyl-hexanoyl, 2-Benzyl-5-methyl-hexanoyl, 2-Benzyl-heptanoyl; Di-Ar-alkanoyl wie 2-Benzyl-3-phenylpropionyl, 2-Benzyl-4-phenylbutyryl, 2-(2-Phenyl-ethyl)-4-phenylbutyryl, 2-(1- oder 2-Naphthylmethyl)-4-phenylbutyryl; Het-alkanoyl wie Morpholinoacetyl, Thiomorpholinoacetyl; Cycloalkylalkanoyl wie Cyclohexylacetyl, 2-oder 3-Cyclohexylpropionyl; FMOC; heterocyclisch substituiertes Arylalkanoyl wie 2-Benzyl-4-(2-, 3- oder 4-pyridyl)- butyryl, 2-Benzyl-4-(2-oxopyrrolidino)-butyryl; N-substituiertes Carbamoyl wie N-Benzylcarbamoyl, N-(2-, 3- oder 4-Pyridylmethyl)-carbamoyl; N,N-disubstituiertes Carbamoyl wie N,N-Dibenzyl-carbamoyl, N-Benzyl-N-2-phenylethyl-carbamoyl, N-Benzyl-N-butyl-carbamoyl, N,N-Bis-2-phenylethyl-carbamoyl, N-2-Morpholinoethyl-N-1-naphthylmethyl-carbamoyl, MC, Pyrrolidinocarbonyl, Piperidinocarbonyl; durch Alkoxycarbonyl substituiertes Arylalkanoyl wie 2-Benzyl-3-ethoxy-carbonyl-propionyl, 2-(1-Naphthylmethyl)-3-ethoxycarbonyl-propionyl; durch substituiertes Carbamoyl substituiertes Arylalkanoyl wie 2-Benzyl-3-(N-isopropylcarbamoyl)-propionyl, 2-(1-Naphthylmethyl)-3-(N-isopropylcarbamoyl)-propionyl, 2-(1-Naphthylmethyl)-3-(N-2-phenylethylcarbamoyl)-propionyl, 2-Benzyl-3-piperidinocarbonyl-propionyl, 2-Benzyl-3-morpholinocarbonyl-propionyl, 2-(1-Naphthylmethyl)-3-morpholinocarbonyl-propionyl; durch Acyl substituiertes Arylalkanoyl wie 2-Benzyl-4-oxo-hexanoyl, 2-Benzyl-4-oxo-5,5-dimethylhexanoyl, 2-Benzyl-4-oxo-6-methylheptanoyl, 2-Benzyl-4-oxo-4-phenyl-butyryl, 2-Benzyl-4-oxo-4-(2-, -(3- oder -(4-pyridyl)-butyryl; Alkylsulfonyl wie Propylsulfonyl.

Besonders bevorzugte Reste X sind H, BOC, MC und Morpholinoacetyl, ferner POA, 4-Phenylbutyryl, 2-Benzyl-butyryl, 2-Benzyl-hexanoyl, 2-Benzyl-heptanoyl, 2-Benzyl-3-phenylpropionyl, 2-Benzyl-4-phenylbutyryl, 2-(2-Phenylethyl)-4-phenyl-butyryl, 2-(1-Naphthylmethyl)-4-phenylbutyryl, CBZ, N-Benzyl-carbamoyl, N-(2-Pyridylmethyl)-carbamoyl, N,N-Dibenzyl-carbamoyl, 2-(1-Naphthylmethyl)-3-ethoxycarbonyl-propionyl und 2-Benzyl-4-oxo-4-(2-pyridyl)butyryl.

V ist bevorzugt -NH-.

Z bedeutet vorzugsweise -CH₂-, -CH₂-O-. -CH₂-NR⁹- (insbesondere -CH₂-NH-) oder -CH₂-S-

Dementsprechend bedeutet die Gruppe -Z-(CH₂)ₐ-CO- bevorzugt -CH₂CH₂CO-, -CH₂-O-CH₂CO-, -CH₂-Gly-, -CH₂-NH-CO- oder -CH₂-S-CH₂-CO-.

Die Gruppe W bedeutet vorzugsweise -NH-CHR⁴-CHOH-CH₂-CO-, insbesondere -NH-CH-(Cyclohexylmethyl)-CHOH-CH₂-CO- ("AHCP" abgeleitet von 4-Amino-3-hydroxy-5-cyclohexylpentansäure), ferner -NH-CH(CH₂CH₂-Cyclohexyl)-CHOH-CH₂-CO- ("AHCH"; abgeleitet von 4-Amino-3-hydroxy-6-cyclohexyl-hexansäure), -NH-CH-(Isobutyl)-CHOH-CH₂-CO- ("Sta"; abgeleitet von Statin) oder -NH-CH(Benzyl)-CHOH-CH₂-CO ("AHPP"; abgeleitet von 4-Amino-3-hydroxy-5-phenylpentansäure). Die Gruppe W bedeutet ferner bevorzugt -NH-CHR⁴-CH(NH₂)-CH₂-CO-, insbesondere -NH-CH(Cyclohexylmethyl)-CH(NH₂)-CH₂-CO- ("DACP"; abgeleitet von 3,4-Diamino-5-cyclohexylpentansäure), -NH-CH(CH₂CH₂-Cyclohexyl)-CH(NH₂)-CH₂-CO-("DACH"; abgeleitet von 3,4-Diamino-6-cyclohexylhexansäure), -NH-CH-(Isobutyl)-CH(NH₂)-CH₂-CO- ("DAMH"; abgeleitet von 3,4-Diamino-6-methylheptansäure) oder -NH-CH(Benzyl)-CH(NH₂)-CH₂-CO- ("DAPP"; abgeleitet von 3,4-Diamino-5-phenylpentansäure) Weiterhin sind von bevorzugter Bedeutung die 2-Alkylderivate von Homologen dieser Gruppen, insbesondere die 2-Isopropyl-derivate, z.B. -NH-CH-(Cyclohexylmethyl)-CHOH-CH₂-CH(Isopropyl)-CO-("2-iPr-homo-AHCP") oder -NH-CH(Isobutyl)-CHOH-CH₂-CH (Isopropyl)-CO-("2-iPr-homo-Sta")

Die Gruppe W besitzt mindestens ein chirales Zentrum. Die Verbindungen der Formel I können daher in verschiedenen - optisch-inaktiven oder optisch-aktiven - Formen vorkommen. Die Formel I umschließt alle diese Formen. Falls W -NH-CHR$^4$-CR$^5$-CH$_2$-CO- und R$^5$ (H, OH) oder (H, NH$_2$) bedeuten, sind die 3S-Hydroxy-4S-amino-Enantiomeren bzw. 3S,4S-Diamino-Enantiomeren bevorzugt. Wenn bei der Bezeichnung von Einzelsubstanzen nichts anderes angegeben ist, beziehen sich die Abkürzungen AHCP, AHCH, Sta, AHPP, DACP, DACH, DAMH und DAPP immer auf diese 3S,4S-Formen.

Die oben erwähnten substituierten Cycloalkyl- und Phenylgruppen tragen vorzugsweise 1-3, insbesondere 1 oder 2 Substituenten.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ij ausgedrückt werden, die der Formel I entsprechen, worin jedoch

in Ia    V-CHR$^2$    -NH-CH(CH$_2$C$_6$H$_5$)- bedeutet;

in Ib    -Z-(CH$_2$)$_a$-CO-    -CH$_2$CH$_2$CO-, -CH$_2$-Gly- oder -CH$_2$-S-CH$_2$-CO- bedeutet;

in Ic    -NR$^3$-CHR$^4$-CR$^5$-(CHR$^6$)$_n$-CO- (= W) AHCP, AHCH, Sta, AHPP, (2-iPr-homo-AHCP), (2-iPr-homo-Sta), DACP, DACH, DAMH oder DAPP bedeutet;

in Id    W AHCP bedeutet;

in Ie    E Ile oder Leu bedeutet;

in If    E Ile bedeutet;

in Ig    Q NH bedeutet;

in Ih    -Q-Y -NH$_2$, -NH-CH$_2$-(3-pyridyl), -NH-CH$_2$-(4-amino-2-methyl-5-pyrimidinyl) oder -NH-CH$_2$-(2-amino-5,6-dimethyl-3-pyrazinyl) bedeutet;

in Ii    V-CHR$^2$    -NH-CH(CH$_2$C$_6$H$_5$)-, -Z-(CH$_2$)$_a$-CO-    -CH$_2$CH$_2$CO-, -CH$_2$-Gly- oder -CH$_2$-S-CH$_2$CO-,

W    AHCP,

E    Ile oder Leu und

-Q-Y    -NH$_2$, -NH-CH$_2$-(3-pyridyl), -NH-CH$_2$-(4-amino-2-methyl-5-pyrimidinyl) oder -NH-CH$_2$-(2-amino-5,6-dimethyl-3-pyrazinyl) bedeuten;

in Ij    V-CHR$^2$    -NH-CH(CH$_2$C$_6$H$_5$)-, -Z-(CH$_2$)$_a$-CO-    -CH$_2$CH$_2$CO-, -CH$_2$-Gly- oder -CH$_2$-S-CH$_2$CO-

W    AHCP,

E    Ile und

-Q-Y    -NH$_2$, -NH-CH$_2$-(3-pyridyl), -NH-CH$_2$-(4-amino-2-methyl-5-pyrimidinyl) oder -NH-CH$_2$-(2-amino-5,6-dimethyl-3-pyrazinyl) bedeuten.

Ferner sind bevorzugt Verbindungen der Formeln I' sowie Ia' bis Ij', die den Formeln I sowie Ia bis Ij entsprechen, worin jedoch

X    H, IPOC, BOC, CBZ, PyOC, POA, Hexanoyl, 3,3-Dimethylbutyryl, Heptanoyl, 2-Propylvaleryl, 3-Phenylpropionyl, 4-Phenylbutyryl, 2-Benzylbutyryl, 2-Benzylhexanoyl, 2-Benzylheptanoyl, 2-Benzyl-4-phenyl-butyryl, 2-Benzyl-4-(4-pyridyl)-butyryl, 2-Benzyl-4-(2-oxo-pyrrolidino)-butyryl, Morpholinoacetyl, Thiomorpholinoacetyl, N-Benzyl-carbamoyl, N-2-Pyridylmethylcarbamoyl, N,N-Dibenzyl-carbamoyl, N-Benzyl-N-butyl-carbamoyl, N-Benzyl-N-2-phenylethyl-carbamoyl, N,N-Bis-2-phenylethyl-carbamoyl, N-2-Morpholinoethyl-N-1-naphthylmethyl-carbamoyl, MC, Pyrrolidinocarbonyl, 2-Benzyl-3-ethoxycarbonyl-propionyl, 2-(1-Naphthylmethyl)-3-ethoxycarbonyl-propionyl, 2-(1-Naphthylmethyl)-3-(N-isopropyl-carbamoyl)-propionyl, 2-(1-Naphthylmethyl)-3-morpholinocarbonyl-propionyl, 2-Benzyl-4-oxo-hexanoyl,    2-Benzyl-4-oxo-5,5-dimethylhexanoyl, 2-Benzyl-4-oxo-6-methyl-heptanoyl, 2-Benzyl-4-oxo-4-phenyl-butyryl, 2-Benzyl-4-oxo-4 (2-pyridyl)-butyryl oder Propylsulfonyl bedeutet.

Ferner sind bevorzugt Verbindungen der Formeln I'' sowie Ia'' bis Ij'', die den Formeln I sowie Ia bis Ij entsprechen, worin jedoch

X    H, BOC, IPOC, CBZ, PyOC, POA, 2-Benzylheptanoyl, 2-Benzyl-4-(2-oxopyrrolidino)-butyryl, Morpholinoacetyl, Thiomorpholinoacetyl, N,N-Dibenzyl-carbamoyl, N-Benzyl-N-2-phenylethyl-carbamoyl, MC, Pyrrolidinocarbonyl oder Propylsulfonyl bedeutet.

Insbesondere sind bevorzugt Verbindungen der Formeln I° sowie Ia° bis Ij°, die den Formeln I sowie Ia bis Ij entsprechen, worin jedoch X BOC bedeutet.

Die Verbindungen der Formel I und auch die Ausgangsstoffe zu ihrer Herstellung werden im übrigen nach an sich bekannten Methoden hergestellt, wie sie in der Literatur (z. B. in den Standardwerken wie Houben-Weyl, Methoden der organischen Chemie, Georg-Thieme-Verlag, Stuttgart; ferner EP-A-45665, EP-A-77028, EP-A-77029, EP-A-81783) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

So können die Verbindungen der Formel I erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, z. B. solche, die der Formel I entsprechen, aber an Stelle einer His-Gruppe eine N(im)-$R^{12}$-His-Gruppe (worin $R^{12}$ eine Aminoschutzgruppe bedeutet, z. B. BOM oder DNP) enthalten, oder solche der Formel X-V-CHR$^2$-CHOH-Z-(CH$_2$)$_a$-CO-NR$^3$-CHR$^4$-CH(NHR$^{12}$)-(CHR$^6$)$_n$-CO-E-Q-Y.

Ferner sind Ausgangsstoffe bevorzugt, die an Stelle des H-Atoms einer Hydroxygruppe eine Hydroxyschutzgruppe tragen, z. B. solche der Formel X-V-CHR$^2$-CHOR$^{13}$-Z-(CH$_2$)$_a$-CO-NR$^3$-CHR$^4$-CHOR$^{13}$-(CHR$^6$)$_n$-CO-E-Q-Y, worin einer oder beide der Reste $R^{13}$ Hydroxyschutzgruppen bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, konnen sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls duchgeführt worden ist. Typisch für solche Gruppen sind insbesondere unsubstituierte oder substituierte Acyl-, Aryl- (z. B. DNP), Aralkoxymethyl- (z. B. BOM) oder Aralkylgruppen (z. B. Benzyl, 4-Nitrobenzyl, Triphenylmethyl).

Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-Jodethoxycarbonyl; Aralkyloxycarbonyl wie CBZ, 4-Methoxybenzyloxycarbonyl, FMOC. Bevorzugte Aminoschutzgruppen sind DNP, BOM, CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl und Acetyl, wobei Benzyl und Acetyl besonders bevorzugt sind.

Es ist ferner möglich, daß Ausgangsstoffe verwendet werden, in denen eine Amino- und eine Hydroxygruppe durch eine gemeinsame Schutzgruppe geschützt sind. So führt z. B. eine Hydrolyse von Verbindungen, die an Stelle der Gruppe X-V-CHR$^2$-CHOH- eine 2,2-Dialkyl- (vorzugsweise 2,2-Dimethyl-) 3-X-4-$R^2$-5-oxazolidinyl-gruppe enthalten, zu Verbindungen der Formel I, worin V = NH ist.

Die als Ausgangsstoffe zu verwendenden funktionellen Derivate der Verbindungen der Formel I können nach üblichen Methoden der Aminosäure- und Peptidsynthese hergestellt werden, wie sie z. B. in den genannten Standardwerken und Patentanmeldungen beschrieben sind.

Das In-Freiheit-Setzen der Verbindungen der Formel I aus ihren funktionellen Derivaten gelingt - je nach der benutzten Schutzgruppe - z. B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran (THF) oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9 : 1. Die Reaktionstemperaturen für die Spaltung liegen zweckmäßig zwischen etwa 0 und etwa 50°, vorzugsweise arbeitet man zwischen 15 und 30° (Raumtemperatur).

Die BOC-Gruppe kann z. B. bevorzugt mit 40 %iger Trifluoressigsäure in Dichlormethan oder mit etwa 3 bis 5 n HCl in Dioxan bei 15-30° abgespalten werden, die FMOC-Gruppe mit einer etwa 5- bis 20%igen Lösung von Dimethylamin, Diethylamin oder Piperidin in DMF bei 15-30°. Eine Abspaltung der DNP-Gruppe gelingt z. B. auch mit einer etwa 3- bis 10%igen Lösung von 2-Mercaptoethanol in DMF/Wasser bei 15-30°. Die genannten Oxazolidinderivate können bevorzugt mit Salzsäure in THF/Wasser/Essigsäure bei 15-30° gespalten werden.

Hydrogenolytisch entfernbare Schutzgruppen (z. B. BOM, CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5-bis 10%igem Pd-C in Methanol bei 20-30°.

Verbindungen der Formel I können auch durch direkte Peptidsynthese aus einer Carbonsäure- und einer Aminkomponente erhalten werden. Als Carbonsäurekomponenten eignen sich z. B. solche der Teilformeln (a) $X-V-CHR^2-CHOH-Z-(CH_2)_a-COOH$, (b) $X-V-CHR^2-CHOH-Z-(CH_2)_a-CO-W-OH$, (c) $X-V-CHR^2-CHOH-Z-(CH_2)_a-CO-W-E^1-OH$ oder (d) $X-V-CHR^2-CHOH-Z-(CH_2)_a CO-W-E-OH$, als Aminkomponenten solche der Teilformeln (a) $H-W-E-Q-Y$, (b) $H-E-Q-Y$, (c) $H-E^2-Q-Y$ oder (d) $H-Q-Y$. Im Fall (c) wird die Peptidbindung innerhalb der Gruppe E geknüpft, falls E aus zwei der angegebenen Aminosäurereste besteht; dabei wird eine Carbonsäure mit einem Amin der jeweils unter (c) angegebenen Formeln umgesetzt, wobei $E^1$ und $E^2$ jeweils einen der bei der Definition von E angegebenen Aminosäurereste bedeutet und $E^1 + E^2 = E$ ist. Dabei arbeitet man zweckmäßig nach üblichen Methoden der Peptid-Synthese, wie sie z. B. in Houben-Weyl, 1.c., Band 15/II, Seiten 1 bis 806 (1974) beschrieben sind.

Die Reaktion gelingt vorzugsweise in Gegenwart eines Dehydratisierungsmittels, z. B. eines Carbodiimids wie DCCI oder Dimethylaminopropylethyl-carbodiimid, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z. B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle von II bzw. III können auch geeignete reaktionsfähige Derivate dieser Stoffe in die Reaktion eingesetzt werden, z. B. solche, in denen reaktive Gruppen intermediär durch Schutzgruppen blockiert sind. Die Aminosäurederivate III können z. B. in Form ihrer aktivierten Ester verwendet werden, die zweckmäßig in situ gebildet werden, z. B. durch Zusatz von HOBt oder N-Hydroxysuccinimid.

Die Ausgangsstoffe der Formeln II und III sind größtenteils bekannt. Sofern sie nicht bekannt sind, können sie nach bekannten Methoden, z. B. den oben angegebenen Methoden der Peptidsynthese und der Abspaltung von Schutzgruppen, hergestellt werden.

Eine bevorzugte Herstellungsweise für die Vebindungen der Formel I ist die Reduktion entsprechender Ketoverbindungen der Formel IV, die ihrerseits z.B. durch "Peptidsynthese" aus Ketocarbonsäuren der Formeln (a) $X-V-CHR^2-CO-Z-(CH_2)_a-COOH$, (b) $X-V-CHR^2-CO-Z-(CH_2)_a-CO-W-OH$, (c) $X-V-CHR^2-COZ-(CH_2)_a-CO-W-E^1-OH$ bzw. (d) $X-V-CHR^2-CO-Z-(CH_2)_a-CO-W-E-OH$ mit Verbindungen der Formeln (a) $H-W-E-Q-Y$, (b) $H-E-Q-Y$, (c) $H-E^2-Q-Y$ bzw. (d) $H-Q-Y$ oder auch durch In-Freiheit-Setzen aus ihren funktionellen Derivaten hergestellt werden können. Die Ketocarbonsäuren ihrerseits können z.B. aus den Säuren der Formel $X-V-CHR^2-COOH$ hergestellt werden, die leicht mit $CH_2N_2/HBr$ in entsprechende Bromketone der Formel $X-V-CHR^2-CO-CH_2Br$ übergeführt werden können. Diese können dann, z.B. mit reaktionsfähigen Derivaten der Malonsäure, der Thioglykolsäure oder des Glycins, weiter reagieren.

Eine Reduktion der Ketoverbindungen der Formel IV gelingt vorzugsweise mit komplexen Metallhydriden wie $NaBH_4$, $LiBH_4$, $KBH_4$, die nicht gleichzeitig die Peptid-Carbonylgruppen reduzieren, in Gegenwart eines inerten Lösungsmittels, z.B. eines Alkohols wie Methanol, Ethanol, Isopropanol, oder Lösungsmittelgemischs, z.B. Methanol/Wasser, bei Temperaturen zwischen etwa -10 und +30°. Auch eine katalytische Hydrierung ist möglich, besonders, wenn das Ausgangsmaterial IV keine S-Atome und keine hydrogenolysierbaren Gruppen wie CBZ enthält. Man hydriert zweckmäßig an einem Edelmetallkatalysator wie Pd-Kohle oder an Raney-Nickel in einem inerten Lösungsmittel, z.B. einem Alkohol wie Methanol, bei Temperaturen zwischen etwa 0 und 30° und Drucken zwischen etwa 1 und 200 bar.

Gewünschtenfalls kann in einer Verbindung der Formel I eine funktionell abgewandelte Amino- und/oder Hydroxygruppe durch Solvolyse oder Hydrogenolyse nach einer der oben beschriebenen Methoden in Freiheit gesetzt werden.

So kann insbesondere eine Verbindung der Formel I, worin X von H verschieden ist, in eine Verbindung der Formel I (X = H) umgewandelt werden, zweckmäßig durch Hydrogenolyse, falls X = CBZ ist, sonst durch selektive Solvolyse. Falls X = BOC ist, kann man z. B. mit HCl in Dioxan bei Raumtemperatur die BOC-Gruppe abspalten.

Andererseits kann man in einer Verbindung der Formel I gegebenenfalls vorhandene freie Amino- und/oder Hydroxygruppen acylieren, vorzugsweise nach einer der oben für die Umsetzung der Verbindungen der Formeln II und III beschriebenen Methoden. Insbesondere kann eine Verbindung der Formel I, worin X-V- HO oder H-NR$^8$- bedeutet, mit einer Säure der Formel X-OH (worin X von H verschieden ist) oder einem ihrer funktionellen Derivate nach einer dieser Methoden acyliert werden.

In einer Verbindung der Formel I kann auch eine Ketogruppe zu einer CHOH-Gruppe reduziert werden, beispielsweise unter den oben für die Reduktion von IV angegebenen Bedingungen.

Eine Ketogruppe in einer Verbindung der Formel I kann auch durch reduktive Aminierung in eine CH(NH$_2$)-Gruppe umgewandelt werden. Man kann ein- oder mehrstufig reduktiv aminieren. So kann man z. B. die Ketoverbindung mit Ammoniumsalzen, z. B. Ammoniumacetat, und NaCNBH$_3$ behandeln, vorzugsweise in einem inerten Lösungsmittel, z. B. einem Alkohol wie Methanol bei Temperaturen zwischen etwa 0 und 50°, insbesondere zwischen 15 und 30°. Weiterhin ist es möglich, die Ketoverbindung zunächst mit Hydroxylamin in üblicher Weise in das Oxim zu überführen und dieses, z. B. durch katalytische Hydrierung an Raney-Nickel, zum Amin zu reduzieren.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z. B. Pikrate, können zur Isolierung und/oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z. B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z. B. Fluorchlor-kohlenwasserstoffen) enthalten. Zweckmäßig ver wendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z. B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-163237 beschriebenen Verbindungen verabreicht, vorzugsweise in Dosierungen zwischen etwa 10 mg und 10 g, insbesondere zwischen 50 mg und 5 g pro Dosierungseinheit. Die tägliche Dosierung liegt vorzugsweise zwischen etwa 0,5 und 600 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Renin-abhängige Hypertonie und Hyperaldosteronismus können wirksam durch Verabreichung von Dosen zwischen etwa 10 und 300 mg/kg Körpergewicht behandelt werden. Für diagnostische Zwecke können die neuen Aminosäurederivate in Einzeldosen zwischen etwa 0,1 und 10 mg/kg Körpergewicht verabfolgt werden.

Vor- und nachstehend sind alle Temperaturen in °C angegeben. In den nachfolgenden Beispielen bedeutet "übliche Aufarbeitung": Man gibt, falls erforderlich, Wasser hinzu, neutralisiert, extrahiert mit Ether oder Dichlormethan, trennt ab, trocknet die organische Phase über Natriumsulfat, filtriert, dampft ein und reinigt durch Chromatographie an Kieselgel und/oder Kristallisation.

Beispiel 1

Ein Gemisch von 950 mg [3S-Hydroxy-4S-(N-(2(S,R)-hydroxy-3S-tert.-butoxycarbonylamino-4-phenyl-butyl-glycyl-amino)-5-cyclohexyl-pentanoyl]-L-isoleucyl-N(imi)-(2,4-dinitrophenyl)-L-histidyl-amid ["N-(2(SR)-Hydroxy-3S-BOC-amino-4-phenyl-butyl)-Gly-AHCP-Ile-(imi-DNP-His)-NH₂"; erhältlich durch Abspaltung der BOC-Gruppe aus BOC-Ile-(imi-DNP-His)-NH₂ mit 4 n HCl in Dioxan, Reaktion mit BOC-AHCP-OH/DCCI/HOBt zu BOC-AHCP-Ile-(imi-DNP-His)-NH₂, erneute Abspaltung der BOC-Gruppe, Reaktion mit N-(4-Benzyl-3-BOC-2,2-dimethyl-5-oxazolidinyl-methyl)-Gly-OH in Gegenwart von N-Methylmorpholin/HOBt/DCCI und Spaltung des Oxazolidinrings analog Beispiel 3], 860 mg 2-Mercaptoethanol, 10 ml DMF und 10 ml Wasser wird unter Rühren bei 20° mit wässeriger Na₂CO₃-Lösung auf pH 8 eingestellt und 2 Std. bei 20° gerührt. Nach üblicher Aufarbeitung erhält man [3S-Hydroxy-4S-(N-2(S,R)-hydroxy-3S-tert.-butoxycarbonylamino-4-phenyl-butyl)-glycyl-amino)-5-cyclohexylpentanoyl]-L-isoleucyl-L-histidyl-amid ("N-(2(S,R)-Hydroxy-3S-BOC-amino-4-phenyl-butyl)-Gly-AHCP-Ile-His-NH₂").

Beispiel 2

Man löst 1 g N-(2(S,R)-Hydroxy-3S-BOC-amino-4-phenylbutyl)-Gly-AHCP-Ile-(imi-Bom-His)-NH₂ [erhältlich über N-(4-Benzyl-3S-BOC-2,2-dimethyl-5-oxazolidinyl-methyl)-Gly-OH und H-AHCP-Ile-(imi-BOM-His)-NH₂] in 10 ml Methanol, hydriert an 0,5%ig. Pd-C bei 20° und 1 bar 3 Std., filtriert, dampft ein und erhält nach üblicher Aufarbeitung N-(2(S,R)-Hydroxy-3S-BOC-amino-4-phenylbutyl)-Gly-AHCP-Ile-His-NH₂.

Beispiel 3

Ein Gemisch von 1 g N-[4-Benzyl-3-CBZ-2,2-dimethyl-5-oxazolidinyl-methyl)-Gly-AHCP-Ile-(N-4-amino-2-methyl-5-pyrimidinyl-methyl-amid), [erhältlich durch Reduktion von 1-Brom-3-CBZ-amino-4-phenyl-butan-2-on (F. 101°) mit LiAlH (O-tert.-C₄H₉)₃ in Ether zu 1-Brom-3-CBZ-amino-4-phenyl-butan-2-ol (Diastereomerengemisch; chromatographisch trennbar in 2 Isomere, F. 103° und F. 146°), Reaktion mit 2,2-Dimethoxypropan/p-Toluolsulfonsäure in Dichlormethan zu 4-Benzyl-5-brommethyl-3-CBZ-2,2-dimethyl-oxazolidin, Umsetzung mit Gly-tert.-butylester/NaJ/NaHCO₃ in DMF zu N-(4-Benzyl-3-CBZ-2,2-diemthyl-5-oxazolidinyl-methyl)-Gly-tert.-butylester mit CF₃COOH in Dichlormethan und Reaktion der freien Säure mit H-AHCP-Ile-(N-4-amino-2-methyl-5-pyrimidinyl-methylamid)], 60 ml THF, 40 ml Essigsäure und 40 ml 10%iger wässeriger Salzsäure wird 20 Std. bei 20° gerührt. Man rührt in NaHCO₃-Lösung ein, extrahiert mit Dichlormethan, arbeitet weiter wie üblich auf und erhält N-(3S-CBZ-amino-2-(S,R)-hydroxy-4-phenyl-butyl)-Gly-AHCP-Ile-(N-4-amino-2-methyl-5-pyrimidinyl-methyl-amid).

Beispiel 4

Eine Lösung von 4,62 g 1-Amino-2-(H-AHCP-Ile-aminomethyl)-4,5-dimethylpyrazin [erhältlich durch Hydrolyse von BOC-Ile-(N-2-amino-5,6-dimethyl-3-pyrazinylmethylamid) (F. 174°, Zers.) mit 4 n HCl in Dioxan zu H-Ile-(N-2-amino-5,6-dimethyl-3-pyrazinylmethyl-amid), Kondensation mit BOC-AHCP-OH zu BOC-AHCP-Ile-(N-2-amino-5,6-dimethyl-3-pyrazinylmethyl-amid) (F. 175°, Zers.) und Abspaltung der BOC-Gruppe] in 60 ml Dichlormethan wird mit 1,01 g N-Methylmorpholin versetzt. Unter Rühren gibt man 3,85 g 5-Thia-7-(S,R)-hydroxy-8S-BOC-amino-9-phenylnonansäure [erhältlich durch Hydrolyse von 6-(4-Benzyl-3-BOC-2,2-dimethyl-5-oxazolidinyl)-5-thiahexansäure] 1,35 g HOBt und eine Lösung von 2,06 g DCCI in 50 ml Dichlormethan hinzu, rührt 14 Std. bei 2-6°, filtriert den ausgeschiedenen Dicyclohexylharnstoff ab und dampft das Filtrat ein. Man arbeitet wie üblich auf und erhält N-(5-Thia-7(S,R)-hydroxy-8S-BOC-amino-9-phenyl-nonanoyl)--AHCP-Ile-(N-2-amino-5,6-dimethyl-3-pyrazinylmethyl-amid).

Beispiel 5

Analog Beispiel 4 erhält man aus N-(3-Thia-5-(S,R)-hydroxy6S-BOC-amino-7-phenyl-heptanoyl)-AHCP-OH und Isoleucin-(N-2-amino-5,6-dimethyl-3-pyrazinylmethyl-amid) das N-(3-Thia-5(S,R)-hydroxy-6S-BOC-amino-7-phenyl-heptanoyl)-AHCP-Ile-(N-2-amino-5,6-dimethyl-3-pyrazinylmethyl-amid), F. 144-146°.

Analog erhält man:

N-(3-Thia-5(S,R)-hydroxy-6S-BOC-amino-7-phenyl-heptanoyl)-AHCP-Ile-(N-4-amino-2-methyl-5-pyrimidinyl-methyl-amid), F. 135-137° (5S-Hydroxy-Form, F. 114°; 5R-Hydroxy-Form, F.184-185°)

N-(3-Thia-5(S,R)-hydroxy-6S-BOC-amino-7-phenyl-heptanoyl)-AHCH-Ile-(N-4-amino-2-methyl-5-pyrimidinyl-methyl-amid)

N-(3-Thia-5(S,R)-hydroxy-6S-BOC-amino-7-phenyl-heptanoyl)-Sta-Ile- -amino-2-methyl-5-pyrimidinyl-methyl-amid)

N-(3-Thia-5(S,R)-hydroxy-6S-BOC-amino-7-phenyl-heptanoyl)-AHPP-Ile-(N-4-amino-2-methyl-5-pyrimidinyl-methyl-amid)

N-(3-Thia-5(S,R)-hydroxy-6S-BOC-amino-7-phenyl-heptanoyl)-(2-iPr-homo-AHCP)-Ile-(N-4-amino-2-methyl-5-pyrimidinyl-methyl-amid)

N-(3-Thia-5(S,R)-hydroxy-6S-BOC-amino-7-phenyl-heptanoyl)-(2-iPr-homo-Sta)-Ile-(N-4-amino-2-methyl-5-pyrimidinyl-methyl-amid)

N-(3-Thia-5(S,R)-hydroxy-6S-morpholinoacetylamino-7-phenyl-heptanoyl)-AHCP-Ile-(N-4-amino-2-methyl-5-pyrimidinyl-methyl-amid)

N-(3-Thia-5(S,R)-hydroxy-6S-morpholinoacetylamino-7-phenyl-heptanoyl)-Sta-Ile-(N-4-amino-2-methyl-5-pyrimidinyl-methyl-amid)

N-(3-Thia-5(S,R)-hydroxy-6S-morpholinoacetylamino-7-phenyl-heptanoyl)-(2-iPr-homo-AHCP)-Ile-(N-4-amino-2-methyl-5-pyrimidinyl-methyl-amid)

N-(3-Thia-5(S,R)-hydroxy-6S-morpholinoacetylamino-7-phenyl-heptanoyl)-(2-iPr-homo-Sta)-Ile-(N-4-amino-2-methyl-5-pyrimidinyl-methyl-amid)

N-(4(S,R)-Hydroxy-5S-BOC-amino-6-phenyl-hexanoyl)-AHCP-Ile-(N-4-amino-2-methyl-5-pyrimidinyl-methyl-amid), F. 173-176°; Hydrochlorid, F. 136-137° (Zers.) N-(4(S,R)-Hydroxy-5S-BOC-amino-6-phenyl-hexanoyl)-Sta-Ile-(N-4-amino-2-methyl-5-pyrimidinyl-methyl-amid)

N-(4(S,R)-Hydroxy-5S-BOC-amino-6-phenyl-hexanoyl)-(2-iPr-homo-AHCP)-Ile-(N-4-amino-2-methyl-5-pyrimidinyl-methyl-amid)

N-(4(S,R)-Hydroxy-5S-BOC-amino-6-phenyl-hexanoyl)-(2-iPr-homo-Sta)-Ile-(N-4-amino-2-methyl-5-pyrimidinyl-methyl-amid), F. 213°; Hydrochlorid, F. 164-165° (Zers.) ·

N-(4(S,R)-Hydroxy-5S-morpholinoacetylamino-6-phenyl-hexanoyl)-AHCP-Ile-(N-4-amino-2-methyl-5-pyrimidinyl-methyl-amid)

N-(4(S,R)-Hydroxy-5S-morpholinoacetylamino-6-phenyl-hexanoyl)-Sta-Ile-(N-4-amino-2-methyl-5-pyrimidinyl-methyl-amid)

N-(4(S,R)-Hydroxy-5S-morpholinoacetylamino-6-phenyl-hexanoyl)-(2-iPr-homo-AHCP)-Ile-(N-4-amino-2-methyl-5-pyrimidinyl-methyl-amid)

N-(4(S,R)-Hydroxy-5S-morpholinoacetylamino-6-phenyl-hexanoyl)-(2-iPr-homo-Sta)-Ile-(N-4-amino-2-methyl-5-pyrimidinyl-methyl-amid)

N-(2(S,R)-Hydroxy-3S-BOC-amino-4-phenyl-butyl)-Gly-AHCP-Ile-(N-4-amino-2-methyl-5-pyrimidinyl-methyl-amid)

N-(2(S,R)-Hydroxy-3S-BOC-amino-4-phenyl-butyl)-Gly-Sta-Ile-(N-4-amino-2-methyl-5-pyrimidinyl-methyl-amid)

N-(2(S,R)-Hydroxy-3S-BOC-amino-4-phenyl-butyl)-Gly-(2-iPr-homo-AHCP)-Ile-(N-4-amino-2-methyl-5-pyrimidinyl-methyl-amid)

N-(2(S,R)-Hydroxy-3S-BOC-amino-4-phenyl-butyl)-Gly-(2-iPr-homo-Sta)-Ile-(N-4-amino-2-methyl-5-pyrimidinyl-methyl-amid).


Beispiel 6

Analog Beispiel 4 erhält man aus N-(4(S,R)-Hydroxy-5S-BOC-amino-6-phenyl-hexanoyl)-AHCP-OH und H-Ile-(N-4-amino-2-methyl-5-pyrimidinyl-methyl-amid) das N-(4(S,R)-Hydroxy-5S-BOC-amino-6-phenyl-hexanoyl)-AHCP-Ile-(N-4-amino-2-methyl-5-pyrimidinyl-methyl-amid), F. 175°.

Analog erhält man:

N-(4(S,R)-Hydroxy-5S-BOC-amino-6-phenyl-hexanoyl)-AHCP-Abu-(N-4-amino-2-methyl-5-pyrimidinyl-methyl-amid)

N-(4(S,R)-Hydroxy-5S-BOC-amino-6-phenyl-hexanoyl)-AHCP-Ala-(N-4-amino-2-methyl-5-pyrimidinyl-methyl-amid)

N-(4(S,R)-Hydroxy-5S-BOC-amino-6-phenyl-hexanoyl)-AHCP-Cal-(N-4-amino-2-methyl-5-pyrimidinyl-methyl-amid)

N-(4(S,R)-Hydroxy-5S-BOC-amino-6-phenyl-hexanoyl)-AHCP-Leu-(N-4-amino-2-methyl-5-pyrimidinyl-methyl-amid)

N-(4(S,R)-Hydroxy-5S-BOC-amino-6-phenyl-hexanoyl)-AHCP-Met-(N-4-amino-2-methyl-5-pyrimidinyl-methyl-amid)

N-(4(S,R)-Hydroxy-5S-BOC-amino-6-phenyl-hexanoyl)-AHCP-Nle-(N-4-amino-2-methyl-5-pyrimidinyl-methyl-amid)

N-(4(S,R)-Hydroxy-5S-BOC-amino-6-phenyl-hexanoyl)-AHCP-Phe-(N-4-amino-2-methyl-5-pyrimidinyl-methyl-amid)

N-(4(S,R)-Hydroxy-5S-BOC-amino-6-phenyl-hexanoyl)-AHCP-Trp-(N-4-amino-2-methyl-5-pyrimidinyl-methyl-amid)

N-(4(S,R)-Hydroxy-5S-BOC-amino-6-phenyl-hexanoyl)-AHCP-Tyr-(N-4-amino-2-methyl-5-pyrimidinyl-methyl-amid)

N-(4(S,R)-Hydroxy-5S-BOC-amino-6-phenyl-hexanoyl)-AHCP-Val-(N-4-amino-2-methyl-5-pyrimidinyl-methyl-amid)

N-(4(S,R)-Hydroxy-5S-BOC-amino-6-phenyl-hexanoyl)-AHCP-Abu-(N-2-amino-5,6-dimethyl-3-pyrazinyl-methyl-amid)

N-(4(S,R)-Hydroxy-5S-BOC-amino-6-phenylhexanoyl)-AHCP-Ala-(N-2-amino-5,6-dimethyl-3-pyrazinyl-methyl-amid)

N-(4(S,R)-Hydroxy-5S-BOC-amino-6-phenyl-hexanoyl)-AHCP-Cal-(N-2-amino-5,6-dimethyl-3-pyrazinyl-methyl-amid)

N-(4(S,R)-Hydroxy-5S-BOC-amino-6-phenyl-hexanoyl)-AHCP-Ile-(N-2-amino-5,6-dimethyl-3-pyrazinyl-methyl-amid), F. 173-176°

N-(4(S,R)-Hydroxy-5S-BOC-amino-6-phenyl-hexanoyl)-AHCP-Leu-(N-2-amino-5,6-dimethyl-3-pyrazinyl-methyl-amid)

N-(4(S,R)-Hydroxy-5S-BOC-amino-6-phenyl-hexanoyl)-AHCP-Met-(N-2-amino-5,6-dimethyl-3-pyrazinyl-methyl-amid)

N-(4(S,R)-Hydroxy-5S-BOC-amino-6-phenyl-hexanoyl)-AHCP-Nle-(N-2-amino-5,6-dimethyl-3-pyrazinyl-methyl-amid)

N-(4(S,R)-Hydroxy-5S-BOC-amino-6-phenyl-hexanoyl)-AHCP-Phe-(N-2-amino-5,6-dimethyl-3-pyrazinyl-methyl-amid)

N-(4(S,R)-Hydroxy-5S-BOC-amino-6-phenyl-hexanoyl)-AHCP-Trp-(N-2-amino-5,6-dimethyl-3-pyrazinyl-methyl-amid)

N-(4(S,R)-Hydroxy-5S-BOC-amino-6-phenyl-hexanoyl)-AHCP-Tyr-(N-2-amino-5,6-dimethyl-3-pyrazinyl-methyl-amid)

N-(4(S,R)-Hydroxy-5S-BOC-amino-6-phenyl-hexanoyl)-AHCP-Val-(N-2-amino-5,6-dimethyl-3-pyrazinyl-methyl-amid).

Beispiel 7

Analog Beispiel 4 erhält man aus N-(3-Thia-5-(S,R)-hydroxy-6S-BOC-amino-7-phenyl-heptanoyl)-AHCP-Ile-OH und 2-Amino-3-aminomethyl-5,6-dimethylpyrazin das N-(3-Thia-5(S,R)-hydroxy-6S-BOC-amino-7-phenyl-heptanoyl)-AHCP-Ile-(N-2-amino-5,6-dimethyl-3-pyrazinyl-methyl-amid), F. 144-146°.

Analog erhält man N-(3-Thia-5(S,R)-hydroxy-6S-BOC-amino-7-phenyl-heptanoyl)-AHCP-Ile-

-(N-3-pyridylmethyl-amid), F. 110-114°
-(N-4-amino-2-methyl-5-pyrimidinyl-methyl-amid), F. 135-137°
-(N-5-tetrazolyl-methyl-amid)
-(N-3-piperidinyl-methyl-amid)
-(N-m-aminomethyl-benzyl-amid)
-(N-p-sulfamoyl-benzyl-amid)
-(p-sulfamoyl-anilid)
-(N-4-methyl-5-imidazolyl-methyl-amid)
-(N-3,5,6-trimethyl-2-pyrazinyl-methyl-amid)
-(N-2,3-dihydroxypropyl-amid).

Beispiel 8

Analog Beispiel 4 erhält man aus N-(3-Thia-5-(S,R)-hydroxy-6S-BOC-amino-7-phenyl-heptanoyl)-AHCP-Ile-OH und 3-Pyridin-methanol den N-(3-Thia-5(S,R)-hydroxy-6S-BOC-amino-7-phenyl-heptanoyl)-AHCP-Ile-(3-pyridyl-methylester).

Beispiel 9

Ein Gemisch von 1,2 g N-(3-Thia-5-oxo-6S-BOC-amino-7-phenyl-heptanoyl)-AHCP-Ile-(N-4-amino-2-methyl-5-pyrimidinyl-methyl-amid) [F 130°; erhältlich durch Reaktion von BOC-Phe-$CH_2$Br (F. 104-105°) mit HS-$CH_2$-COOH in THF in Gegenwart von NaOCH₃ zu BOC-Phe-$CH_2$-S-$CH_2$-COOH (F. 110-111°) und nachfolgende Umsetzung mit H-AHCP-Ile-(N-4-amino-2-methyl-5-pyrimidinyl-amid)], 60 mg NaBH₄ und 20 ml Methanol wird 1 Std. bei 0° gerührt. Nach dem Eindampfen und üblicher Aufarbeitung erhält man N-(3-Thia-5(S.R)-hydroxy-6S-BOC-amino-7-phenyl-heptanoyl)-AHCP-Ile-(N-4-amino-2-methyl-5-pyrimidinyl-methyl-amid), Diastereomerengemisch, F. 135-137°; Hydrochlorid, F. 153-154° (Zers.)

Analog erhält man durch Reduktion der entsprechenden Ketoverbindungen:

N-(3-Thia-5(S,R)-hydroxy-6S-IPOC-amino-7-phenyl-heptanoyl)-Ile-(N-4-amino-2-methyl-5-pyrimidinyl-methyl-amid)

N-(3-Thia-5(S,R)-hydroxy-6S-pyrrolidinocarbonyl-amino-7-phenyl-heptanoyl)-Ile-(N-4-amino-2-methyl-5-pyrimidinyl-methyl-amid)

N-(3-Thia-5(S,R)-hydroxy-6S-MC-amino-7-phenyl-heptanoyl)-Ile-(N-4-amino-2-methyl-5-pyrimidinyl-methyl-amid)

N-(3-Thia-5(S,R)-hydroxy-6S-morpholinoacetyl-amino-7-phenyl-heptanoyl)-Ile-(N-4-amino-2-methyl-5-pyrimidinyl-methyl-amid)

N-(3-Thia-5(S,R)-hydroxy-6S-(3,3-dimethylbutyryl)-amino-7-phenyl-heptanoyl)-Ile-(N-4-amino-2-methyl-5-pyrimidinyl-methyl-amid)

N-(3-Thia-5(S,R)-hydroxy-6S-PyOC-amino-7-phenyl-heptanoyl)-Ile-(N-4-amino-2-methyl-5-pyrimidinyl-methyl-amid)

N-(3-Thia-5(S,R)-hydroxy-6S-propylsulfonyl-amino-7-phenyl-heptanoyl)-Ile-(N-4-amino-2-methyl-5-pyrimidinyl-methyl-amid)

N-(3-Thia-5(S,R)-hydroxy-6S-BOC-amino-7-phenyl-heptanoyl)-AHCP-Ile-(N-2-amino-5,6-dimethyl-3-pyrazinyl-methyl-amid),
F. 144-146°
N-(3-Thia-5(S,R)-hydroxy-6S-IPOC-amino-7-phenyl-heptanoyl)-AHCP-Ile-(N-2-amino-5,6-dimethyl-3-pyrazinyl-methyl-amid),
N-(3-Thia-5(S,R)-hydroxy-6S-pyrrolidinocarbonyl-amino-7-phenyl-heptanoyl)-AHCP-Ile-(N-2-amino-5,6-dimethyl-3-pyrazinyl-methyl-amid),
N-(3-Thia-5(S,R)-hydroxy-6S-MC-amino-7-phenyl-heptanoyl)-AHCP-Ile-(N-2-amino-5,6-dimethyl-3-pyrazinyl-methyl-amid),
N-(3-Thia-5(S,R)-hydroxy-6S-morpholinoacetyl-amino-7-phenyl-heptanoyl)-AHCP-Ile-(N-2-amino-5,6-dimethyl-3-pyrazinyl-methyl-amid),
N-(3-Thia-5(S,R)-hydroxy-6S-(3,3-dimethylbutyryl)-amino-7-phenyl-heptanoyl)-AHCP-Ile-(N-2-amino-5,6-dimethyl-3-pyrazinyl-methyl-amid),
N-(3-Thia-5(S,R)-hydroxy-6S-PyOC-amino-7-phenyl-heptanoyl)-AHCP-Ile-(N-2-amino-5,6-dimethyl-3-pyrazinyl-methyl-amid),
N-(3-Thia-5(S,R)-hydroxy-6S-propylsulfonyl-amino-7-phenyl-heptanoyl)-AHCP-Ile-(N-2-amino-5,6-dimethyl-3-pyrazinyl-methyl-amid),

N-[3-Thia-5(S,R)-hydroxy-6S-BOC-amino-7-(1-naphthyl)-heptanoyl]-AHCP-Ile-(N-2-amino-5,6-dimethyl-3-pyrazinyl-methyl-amid),
N-(3-Thia-5(S,R)-hydroxy-6S-BOC-amino-7-cyclohexyl-heptanoyl)-AHCP-Ile-(N-2-amino-5,6-dimethyl-3-pyrazinyl-methyl-amid),
N-(3-Thia-5(S,R)-hydroxy-6S-BOC-amino-nonanoyl)-AHCP-Ile-(N-2-amino-5,6-dimethyl-3-pyrazinyl-methyl-amid),
N-(3-Thia-5(S,R)-hydroxy-6S-BOC-amino-decanoyl)-AHCP-Ile-(N-2-amino-5,6-dimethyl-3-pyrazinyl-methyl-amid),
N-[3-Thia-5(S,R)-hydroxy-6S-BOC-amino-7-(3-indolyl)-heptanoyl]-AHCP-Ile-(N-2-amino-5,6-dimethyl-3-pyrazinyl-methyl-amid),

N-(4(SR)-Hydroxy-5S-BOC-amino-6-phenyl-hexanoyl)-AHCP-Ile-(N-4-amino-2-methyl-5-pyrimidinyl-methyl-amid, F. 175°
N-(4(SR)-Hydroxy-5S-IPOC-amino-6-phenyl-hexanoyl)-AHCP-Ile-(N-4-amino-2-methyl-5-pyrimidinyl-methyl-amid
N-(4(SR)-Hydroxy-5S-pyrrolidinocarbonyl-amino-6-phenyl-hexanoyl)-AHCP-Ile-(N-4-amino-2-methyl-5-pyrimidinyl-methyl-amid

N-(4(SR)-Hydroxy-5S-MC-amino-6-phenyl-hexanoyl)-AHCP-Ile-(N-4-amino-2-methyl-5-pyrimidinyl-methyl-amid
N-(4(SR)-Hydroxy-5S-morpholinoacetyl-amino-6-ph   -hexanoyl)-AHCP-Ile-(N-4-amino-2-methyl-5-pyrimidinyl-methyl-amid
N-(4(SR)-Hydroxy-5S-(3,3-dimethylbutyryl)-amino-6-phenyl-hexanoyl)-AHCP-Ile-(N-4-amino-2-methyl-5-pyrimidinyl-methyl-amid
N-(4(SR)-Hydroxy-5S-PyOC-amino-6-phenyl-hexanoyl)-AHCP-Ile-(N-4-amino-2-methyl-5-pyrimidinyl-methyl-amid
N-(4(SR)-Hydroxy-5S-propylsulfonyl-amino-6-phenyl-hexanoyl)-AHCP-Ile-(N-4-amino-2-methyl-5-pyrimidinyl-methyl-amid

N-(4(S,R)-Hydroxy-5S-BOC-amino-6-phenyl-heptanoyl)-AHCP-Ile-(N-2-amino-5,6-dimethyl-3-pyrazinyl-methyl-amid), F. 173-176°
N-(4(S,R)-Hydroxy-5S-IPOC-amino-6-phenyl-heptanoyl)-AHCP-Ile-(N-2-amino-5,6-dimethyl-3-pyrazinyl-methyl-amid)
N-(4(S,R)-Hydroxy-5S-pyrrolidinocarbonyl-amino-6-phenyl-heptanoyl)-AHCP-Ile-(N-2-amino-5,6-dimethyl-3-pyrazinyl-methyl-amid)
N-(4(S,R)-Hydroxy-5S-MC-amino-6-phenyl-heptanoyl)-AHCP-Ile-(N-2-amino-5,6-dimethyl-3-pyrazinyl-methyl-amid)
N-(4(S,R)-Hydroxy-5S-morpholinoacetyl-amino-6-phenyl-heptanoyl)-AHCP-Ile-(N-2-amino-5,6-dimethyl-3-pyrazinyl-methyl-amid)
N-(4(S,R)-Hydroxy-5S-(3,3-dimethylbutyryl)-amino-6-phenyl-heptanoyl)-AHCP-Ile-(N-2-amino-5,6-dimethyl-3-pyrazinyl-methyl-amid)
N-(4(S,R)-Hydroxy-5S-PyOC-amino-6-phenyl-heptanoyl)-AHCP-Ile-(N-2-amino-5,6-dimethyl-3-pyrazinyl-methyl-amid)
N-(4(S,R)-Hydroxy-5S-propylsulfonyl-amino-6-phenyl-heptanoyl)-AHCP-Ile-(N-2-amino-5,6-dimethyl-3-pyrazinyl-methyl-amid)

N-(2(S,R)Hydroxy-3S-BOC-amino-4-phenyl-butyl)-Gly-AHCP-Ile-N-(4-amino-2-methyl-5-pyrimidinyl-methyl-amid)
N-(5(S,R)-Hydroxy-6S-BOC-amino-7-phenyl-heptanoyl)-AHCP-Ile-N-(4-amino-2-methyl-5-pyrimidinyl-methyl-amid)
N-(4(S,R)-Hydroxy-5S-BOC-amino-6-phenyl-2-hexenoyl)-AHCP-Ile-N-(4-amino-2-methyl-5-pyrimidinyl-methyl-amid)
N-(4(S,R)-Hydroxy-5S-BOC-amino-6-phenyl-2-hexinoyl))-AHCP-Ile-N-(4-amino-2-methyl-5-pyrimidinyl-methyl-amid)

N-(3-Thia-5(S,R)-hydroxy-6S-BOC-amino-7-phenyl-heptanoyl)-DACP-Ile-N-(4-amino-2-methyl-5-pyrimidinyl-methyl-amid)
N-(3-Thia-5(S,R)-hydroxy-6S-BOC-amino-7-phenyl-heptanoyl)-DACH-Ile-N-(4-amino-2-methyl-5-pyrimidinyl-methyl-amid)

N-(3-Thia-5(S,R)-hydroxy-6S-BOC-amino-7-phenyl-heptanoyl)-DAMH-Ile-N-(4-amino-2-methyl-5-pyrimidinyl-methyl-amid)
N-(3-Thia-5(S,R)-hydroxy-6S-BOC-amino-7-phenyl-heptanoyl)-DAPP-Ile-N-(4-amino-2-methyl-5-pyrimidinyl-methyl-amid)

N-(4(S,R)-Hydroxy-5S-BOC-amino-6-phenyl-hexanoyl)-DACP-Ile-N-(4-amino-2-methyl-5-pyrimidinyl-methyl-amid)
N-(4(S,R)-Hydroxy-5S-BOC-amino-6-phenyl-hexanoyl)-DAMH-Ile-N-(4-amino-2-methyl-5-pyrimidinyl-methyl-amid)
N-(2(S,R)-Hydroxy-3S-BOC-amino-4-phenyl-butyl)-Gly-DACP-Ile-N-(4-amino-2-methyl-5-pyrimidinyl-methyl-amid)
N-(2(S,R)-Hydroxy-3S-BOC-amino-4-phenyl-butyl)-Gly-DAMH-Ile-N-(4-amino-2-methyl-5-pyrimidinyl-methyl-amid).

Beispiel 10

Eine Lösung von 1 g N-(3-Thia-5(S,R)hydroxy-6S-BOC-amino-7-phenyl-heptanoyl)-AHCP-Ile-(N-3-pyridyl-methyl-amid) in 20 ml 4 n HCl in Dioxan wird 30 Min. bei 20° gerührt und dann eingedampft. Man erhält das Hydrochlorid von N-(3-Thia-5(S,R)-hydroxy-6S-amino-7-phenyl-heptanoyl)-AHCP-Ile-(N-3-pyridyl-methyl-amid).

Analog erhält man die Hydrochloride von
N-(3-Thia-5(S,R)-hydroxy-6S-amino-7-phenyl-heptanoyl)-AHCP-Ile-(N-4-amino-2-methyl-5-pyrimidinyl-methyl-amid)
N-(3-Thia-5(S,R)-hydroxy-6S-amino-7-phenyl-heptanoyl)-AHCP-Ile-(N-2-amino-5,6-dimethyl-3-pyrazinyl-methyl-amid)
N-(4-(S,R)-Hydroxy-5S-amino-6-phenyl-hexanoyl)-AHCP-Ile-(N-4-amino-2-methyl-5-pyrimidinyl-methyl-amid)
N-(4-(S,R)-Hydroxy-5S-amino-6-phenyl-hexanoyl)-AHCP-Ile-(N-2-amino-5,6-dimethyl-3-pyrazinyl-methyl-amid)

Beispiel 11

Man löst 1 g CBZ-(N-(4(S,R)-Hydroxy-5S-CBZ-amino-6-phenyl-hexanoyl)-AHCP-Ile-NH₂ in 10 ml Ethanol, hydriert an 0,5 g 10 %igem Pd-C bei 20° und 1 bar bis zum Stillstand, filtriert, dampft ein und erhält nach chromatographischer Reinigung N-(4(S,R)-Hydroxy-5S-amino-6-phenyl-hexanoyl)-AHCP-Ile-NH₂.

Beispiel 12

Analog Beispiel 11 erhält man aus N-[3S-CBZ-amino-4S-(4(S,R)-hydroxy-5S-BOC-amino-6S-phenyl-hexanoyl-amino)-5-cyclohexyl-pentanoyl]-Ile-NH₂ das 3S-(4(S,R)-hydroxy-5S-amino-6-phenyl-hexanoyl-amino)-DACP-Ile-NH₂.

Beispiel 13

a) Analog Beispiel 4 erhält man durch Reaktion von 4-(4-Benzyl-3-BOC-2,2-dimethyl-5-oxazolidinyl)-buttersäure mit (3-Oxo-4S-amino-5-cyclohexylpentanoyl)-Ile-NH₂ [erhältlich durch Reaktion von 2S-BOC-amino-3-cyclohexylpropionsäure mit Carbonyldiimidazol zum Imidazolid, Umsetzung mit Malonsäure-monomethylester zu 3-Oxo-4S-BOC-amino-5-cyclohexylpentansäuremethylester, Verseifung, Kondensation mit H-Ile-NH₂ zu (3-Oxo-4S-BOC-amino-5-cyclohexylpentanoyl)-Ile-NH₂ und Abspaltung der BOC-Gruppe und nachfolgende Spaltung des Oxazolidinrings] das [3-Oxo-4S-(5-(S,R)-hydroxy-6S-BOC-amino-7-phenyl-heptanoyl)-amino -5-cyclohexylpentanoyl]-Ile-NH₂.

b) Eine Lösung von 10 g des vorstehenden Amids in 250 ml CH₃OH wird an 1 g 10 %ig. Pd-C bei 20° und 1 bar bis zum Stillstand hydriert. Nach Filtrieren und Eindampfen erhält man ein Gemisch von [3R- und [3S-Hydroxy-4S-((5(S,R)-hydroxy-6S-BOC-amino-7-phenyl-heptanoyl)-amino)-5-cyclohexylpentanoyl]-Ile-NH₂, das chromatographisch getrennt werden kann.

Beispiel 14

Eine Lösung von 454 mg [3-Oxo-4S-(N-(5(S,R)-Hydroxy-6S-amino-7-phenyl-heptanoyl)-amino)-5-cyclohexylpentanoyl]-Ile-NH₂ und 1,43 g Na₂CO₃ . 10 H₂O in 5 ml Methanol und 5 ml Wasser wird mit 70 mg Hydroxylaminhydrochlorid versetzt und 14 Std. bei 20° gerührt. Das ausgefallene Oxim wird abfiltriert, getrocknet, in 10 ml Methanol gelöst und an 0,5 g Raney-Ni bei 20° und 5 bar hydriert. Man filtriert, dampft das Filtrat ein und erhält nach üblicher Aufarbeitung (N-(5(S,R)-Hydroxy-6S-amino-7-phenyl-heptanoyl)-DACP-Ile-NH₂ als Diastereomerengemisch, das chromatographisch getrennt werden kann.

Beispiel 15

Analog Beispiel 4 erhält man durch Reaktion der entsprechenden 3-Thia-5(S,R)-hydroxy-6S-acylamino-7-phenyl-heptansäuren mit H-AHCP-Ile-(N-4-amino-2-methyl-5-pyrimidinylmethyl-amid) die nachstehenden    [3-Thia-5(S,R)-hydroxy-6S-acylamino-7-phenyl-heptanoyl-AHCP-Ile-(N-4-amino-2-methyl-5-pyrimidinylmethyl-amide):

6S-BOC-amino-
6S-BOC-Gly-amino-
6S-(4-BOC-amino-butyryl-amino)-
6S-(6-BOC-amino-hexanoyl-amino)-, F. 153°
6S-(8-BOC-amino-octanoyl-amino)-
6S-(N-BOC-N-methyl-Gly-amino)-
6S-(4-Dimethylamino-butyryl-amino)-, F. 166-167°;
Dihydrochlorid, F. 185-186° (Zers.)
6S-(6-Dimethylamino-hexanoyl-amino)-
6S-(1-Imidazolyl-acetyl-amino)-
6S-[3-(1-Imidazolyl)-propionyl-amino]-
6S-(3-Piperidino-propionyl-amino)-
6S-[N-(6-BOC-amino-hexyl)-carbamoyl]-amino-
6S-[N-(3-Dimethylamino-propyl)-carbamoyl]-amino-
6S-[N-(6-Dimethylamino-hexyl)-carbamoyl]-amino-
6S-(4-BOC-amino-piperidinocarbonyl-amino)-.

Analog erhält man die nachstehenden [4(S,R)-Hydroxy-5S-acylamino-6-phenyl-hexanoyl]-AHCP-Ile-(N-4-amino-2-methyl-5-pyrimidinyl-methyl-amide):

6S-BOC-amino-
6S-BOC-Gly-amino-
6S-(4-BOC-amino-butyryl-amino)-
6S-(6-BOC-amino-hexanoyl-amino)-
6S-(8-BOC-amino-octanoyl-amino)-
6S-(N-BOC-N-methyl-Gly-amino)-
6S-(4-Dimethylamino-butyryl-amino)-
6S-(6-Dimethylamino-hexanoyl-amino)-
6S-(1-Imidazolyl-acetyl-amino)-
6S-[3-(1-Imidazolyl)-propionyl-amino]-
6S-(3-Piperidino-propionyl-amino)-
6S-[N-(6-BOC-amino-hexyl)-carbamoyl]-amino-
6S-[N-(3-Dimethylamino-propyl)-carbamoyl]-amino-
6S-[N-(6-Dimethylamino-hexyl)-carbamoyl]-amino-
6S-(4-BOC-amino-piperidinocarbonyl-amino)-;

die    nachstehenden    [3-Thia-5(S,R)-hydroxy-6S-acylamino-7-phenyl-heptanoyl]-AHCP-Ile-(N-2-amino-5,6-dimethyl-3-pyrazinylmethyl-amide):

6S-BOC-amino-
6S-BOC-Gly-amino-
6S-(4-BOC-amino-butyryl-amino)-
6S-(6-BOC-amino-hexanoyl-amino)-
6S-(8-BOC-amino-octanoyl-amino)-
6S-(N-BOC-N-methyl-Gly-amino)-
6S-(4-Dimethylamino-butyryl-amino)-
6S-(6-Dimethylamino-hexanoyl-amino)-
6S-(1-Imidazolyl-acetyl-amino)-
6S-[3-(1-Imidazolyl)-propionyl-amino]-
6S-(3-Piperidino-propionyl-amino)-
6S-[N-(6-BOC-amino-hexyl)-carbamoyl]-amino-
6S-[N-(3-Dimethylamino-propyl)-carbamoyl]-amino-

6S-[N-(6-Dimethylamino-hexyl)-carbamoyl]-amino-
6S-(4-BOC-amino-piperidinocarbonyl-amino)-;

die nachstehenden [4(S,R)-Hydroxy-5S-acylamino-6-phenyl-hexanoyl]-AHCP-Ile-(N-2-amino-5,6-dimethyl-3-pyrazinyl-methyl-amide):

6S-BOC-amino-
6S-BOC-Gly-amino-
6S-(4-BOC-amino-butyryl-amino)-
6S-(6-BOC-amino-hexanoyl-amino)-
6S-(8-BOC-amino-octanoyl-amino)-
6S-(N-BOC-N-methyl-Gly-amino)-
6S-(4-Dimethylamino-butyryl-amino)-
6S-(6-Dimethylamino-hexanoyl-amino)-
6S-(1-Imidazolyl-acetyl-amino)-
6S-[3-(1-Imidazolyl)-propionyl-amino]-
6S-(3-Piperidino-propionyl-amino)-
6S-[N-(6-BOC-amino-hexyl)-carbamoyl]-amino-
6S-[N-(3-Dimethylamino-propyl)-carbamoyl]-amino-
6S-[N-(6-Dimethylamino-hexyl)-carbamoyl]-amino-
6S-(4-BOC-amino-piperidinocarbonyl-amino)-.
sowie    die    nachstehenden    [4(S,R)-Hydroxy-5S-acylamino-6-phenyl-hexanoyl]-AHCP-Ile-(N-3-pyridyl-methyl-amide):

6S-BOC-amino-
6S-BOC-Gly-amino-
6S-(4-BOC-amino-butyryl-amino)-
6S-(6-BOC-amino-hexanoyl-amino)-
6S-(8-BOC-amino-octanoyl-amino)-
6S-(N-BOC-N-methyl-Gly-amino)-
6S-(4-Dimethylamino-butyryl-amino)-
6S-(6-Dimethylamino-hexanoyl-amino)-
6S-(1-Imidazolyl-acetyl-amino)-
6S-[3-(1-Imidazolyl)-propionyl-amino]-
6S-(3-Piperidino-propionyl-amino)-
6S-[N-(6-BOC-amino-hexyl)-carbamoyl]-amino-
6S-[N-(3-Dimethylamino-propyl)-carbamoyl]-amino-
6S-[N-(6-Dimethylamino-hexyl)-carbamoyl]-amino-
6S-(4-BOC-amino-piperidinocarbonyl-amino)-

Beispiel 16

Analog Beispiel 10 erhält man durch Spaltung der entsprechenden BOC-Derivate die nachstehenden [3-Thia-5(S,R)-hydroxy-6S-amino- bzw. -6S-aminoacylamino-7-phenylheptanoyl]-AHCP-Ile-(N-4-amino-2-methyl-5-pyrimidinylmethyl-amide)
6S-Amino-, F. 202°; Hydrochlorid, F 170-171° (Zers.)
6S-H-Gly-amino-
6S-(4-Aminobutyryl-amino)-
6S-(6-Aminohexanoyl-amino)-, Dihydrochlorid. F. 183-184°
6S-(8-Aminooctanoyl)-amino-
6S-(N-Methyl-Gly-amino)-

6S-[N-(6-Aminohexyl)-carbamoyl]-amino-
6S-(4-Amino-piperidinocarbonyl-amino)-;
ferner die nachstehenden [4(S,R)-Hydroxy-5S-
amino- bzw. -5S-amino- bzw. -5S-aminoacylamino-
6-phenylhexanoyl]-AHCP-Ile-(N-4-amino-2-methyl-
5-pyrimidinyl-methyl-amide):

5S-Amino-
5S-H-Gly-amino-
5S-(4-Aminobutyryl-amino)-
5S-(6-Aminohexanoyl-amino)-, F. 192° (Zers.)
5S-(8-Aminooctanoyl-amino)-
5S-(N-Methyl-Gly-amino)-
5S-[N-(6-Aminohexyl)-carbamoyl]-amino-
5S-(4-Amino-piperidinocarbonyl-amino)-;
die nachstehenden [3-Thia-5(S,R)-hydroxy-6S-
acylamino-7-phenyl-heptanoyl]-AHCP-Ile-(N-2-
amino-5,6-dimethyl-3-pyrazinylmethyl-amide):

5S-Amino-
5S-H-Gly-amino-
5S-(4-Aminobutyryl-amino)-
5S-(6-Aminohexanoyl-amino)- .
5S-(8-Aminooctanoyl-amino)-
5S-(N-Methyl-Gly-amino)-
5S-[N-(6-Aminohexyl)-carbamoyl]-amino-
5S-(4-Amino-piperidinocarbonyl-amino)-;

die nachstehenden [4(S,R)-Hydroxy-5S-acylamino-
6-phenyl-hexanoyl]-AHCP-Ile-(N-2-amino-5,6-
dimethyl-3-pyrazinyl-methyl-amide):

5S-Amino-
5S-H-Gly-amino-
5S-(4-Aminobutyryl-amino)-
5S-(6-Aminohexanoyl-amino)-
5S-(8-Aminooctanoyl-amino)-
5S-(N-Methyl-Gly-amino)-
5S-[N-(6-Aminohexyl)-carbamoyl]-amino-
5S-(4-Amino-piperidinocarbonyl-amino)-;

sowie die nachstehenden [4(S,R)-Hydroxy-5S-
acylamino-6-phenyl-hexanoyl]-AHCP-Ile-(N-3-
pyridyl-methyl-amide):

5S-Amino-
5S-H-Gly-amino-
5S-(4-Aminobutyryl-amino)-
5S-(6-Aminohexanoyl-amino)-
5S-(8-Aminooctanoyl-amino)-
5S-(N-Methyl-Gly-amino)-
5S-[N-(6-Aminohexyl)-carbamoyl]-amino-
5S-(4-Amino-piperidinocarbonyl-amino)-.
Die nachstehenden Beispiele betreffen
pharmazeutische Zubereitungen.

Beispiel A: Injektionsgläser

Eine Lösung von 100 g N-(3-Thia-5(S,R)-
hydroxy-6S-BOC-amino-7-phenyl-heptanoyl)-
AHCP-Ile-(N-2-amino-5,6-dimethyl-3-pyrazinyl-
methyl-amid) und 5 g Dinatriumhydrogenphosphat
in 3 l zweifach destilliertem Wasser wird mit 2 n
Salzsäure auf pH 6,5 eingestellt, steril filtriert, in
Injektionsgläser abgefüllt, unter sterilen Bedingungen lyophilisiert und steril verschlossen. Jedes Injektionsglas enthält 500 mg Wirkstoff.

Beispiel B: Suppositorien

Man schmilzt ein Gemisch von 500 g N-(4-
(S,R)-Hydroxy-5S-BOC-amino-6-phenyl-hexanoyl)-
AHCP-Ile-(N-4-amino-2-methyl-5-pyrimidinyl-
methyl-amid) mit 100 g Sojalecithin und 1400 g
Kakaobutter, gießt in Formen und läßt erkalten.
Jedes Suppositorium enthält 500 mg Wirkstoff.

**Ansprüche**

1. Aminosäurederivate der Formel I
$$X\text{-}V\text{-}CHR^2\text{-}CHOH\text{-}Z\text{-}(CH_2)_a\text{-}CO\text{-}NR^3\text{-}CHR^4\text{-}CR^5\text{-}(CHR^6)_n\text{-}CO\text{-}E\text{-}Q\text{-}Y \qquad I$$
worin

X H, $R^1$, $R^1\text{-}O\text{-}C_mH_{2m}\text{-}CO\text{-}$, $R^1\text{-}C_mH_{2m}\text{-}O\text{-}CO\text{-}$, $R^1\text{-}C_mH_{2m}\text{-}CO\text{-}$, $R^1\text{-}SO_2\text{-}$, $(R^1\text{-}C_mH_{2m})\text{-}L(R^7\text{-}C_pH_{2p})\text{-}C_rH_{2r}\text{-}CO\text{-}$, $R^1\text{-}(NHCH_2CH_2)_m\text{-}NH\text{-}CH_2CO\text{-}$, 9-Fluorenyl-$C_mH_{2m}\text{-}O\text{-}CO\text{-}$ oder $(R^1\text{-}C_mH_{2m}\text{-}(T)_x\text{-}CO\text{-}C_rH_{2r})\text{-}L(R^7\text{-}C_pH_{2p})\text{-}C_yH_{2y}\text{-}CO\text{-}$,

V $-CH_2\text{-}$, $-O\text{-}$ oder $-NR^8\text{-}$,

Z $-CH_2\text{-}$, $-CH=CH\text{-}$, $-C\equiv C\text{-}$, $-CH_2\text{-}O\text{-}$, $-CH_2\text{-}NR^9\text{-}$ oder $-CH_2\text{-}S\text{-}$,

E 0 bis 2 peptidartig miteinander verbundene Aminosäurereste ausgewählt aus der Gruppe bestehend aus Abu, Ala, Cal, His, Ile, Leu, Met, Nle, Phe, Trp, Tyr und Val,

Q und T jeweils O oder NH,

Y $-C_tH_{2t}\text{-}R^{10}$ oder $-C_wH_{2w}\text{-}(CR^{11})_s\text{-}C_tH_{2t}\text{-}R^{10}$,

$R^1$, $R^2$, $R^4$, $R^7$ und $R^{10}$ jeweils H, A, Ar, Ar-alkyl, Het, Het-alkyl, unsubstituiertes oder ein- oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

$R^3$, $R^6$, $R^8$ und $R^9$ jeweils H oder A,

$R^5$ und $R^{11}$ jeweils (H, OH), (H, $NH_2$) oder $=O$,

L CH oder N,

a, m, p, r, t, w und y jeweils 0, 1, 2, 3, 4, 5, 6, 7, 8, 9 oder 10,

n und s jeweils 1 oder 2,

x 0 oder 1,

Ar unsubstituiertes oder ein- oder mehrfach durch A, AO, Hal, CF₃, HO, H₂NCO, HANCO, A₂NCO, H₂NSO₂, HANSO₂, A₂NSO₂, Hydroxyalkyl mit 1-8 C-Atomen, H₂N und/oder Aminoalkyl mit 1-8 C-Atomen substituiertes Phenyl oder unsubstituiertes Naphthyl,

Het einen gesättigten oder ungesättigten 5- oder 6-gliedrigen heterocyclischen Rest mit 1-4 N-, O- und/oder S-Atomen, der mit einem Benzolring kondensiert und/oder ein- oder mehrfach durch A, AO, Hal, CF₃, HO, O₂N, Carbonylsauerstoff, H₂N, HAN, A₂N, AcNH, AS, ASO, ASO₂, AOOC, CN, H₂NCO, HOOC, H₂NSO₂, ASO₂NH, Ar, Ar-alkenyl, Hydroxyalkyl und/oder Aminoalkyl mit jeweils 1-8 C-Atomen substituiert sein kann und/oder dessen N- und/oder S-Heteroatome auch oxydiert sein können,

Hal F, Cl, Br oder J,

Ac A-CO-, Ar-CO- oder A-NH-CO- und

A Alkyl mit 1-8 C-Atomen bedeuten,

worin ferner an Stelle einer oder mehrerer -NH-CO-Gruppen auch eine oder mehrere -NA-CO-Gruppen stehen können,

sowie deren Salze.

2. a) N-(3-Thia-5-hydroxy-6S-BOC-amino-7-phenyl-heptanoyl)-AHCP-Ile-(N-3-pyridyl-methyl-amid);

b) N-(4-Hydroxy-5-BOC-amino-6-phenyl-hexanoyl)-AHCP-Ile-(N-2-amino-5,6-dimethyl-3-pyrazinyl-methyl-amid);

c) N-(3-Thia-5-hydroxy-6S-BOC-amino-7-phenyl-heptanoyl)-AHCP-Ile-(N-2-amino-5,6-dimethyl-3-pyrazinyl-methyl-amid);

d) N-(3-Thia-5-hydroxy-6S-BOC-amino-7-phenyl-heptanoyl)-AHCP-Ile-(N-4-amino-2-methyl-5-pyrimidinyl-methyl-amid).

e) N-(4-Hydroxy-5-BOC-amino-6-phenyl-hexanoyl)-AHCP-Ile-(N-4-amino-2-methyl-5-pyrimidinyl-methyl-amid).

3. Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man eine Carbonsäure der Formel II

X-V-CHR²-CHOH-Z-(CH₂)ₐ-G¹-OH    II

worin G¹

(a) -CO-,

(b) -CO-W-,

(c) -CO-W-E¹-,

(d) -CO-W-E- und

W -NR³-CHR⁴-CR⁵-(CHR⁶)ₙ-CO- bedeuten oder eines ihrer reaktionsfähigen Derivate mit einer Verbindung der Formel III

H-G²    III

worin G²

(a) -W-E-Q-Y,

(b) -E-Q-Y,

(c) -E²-Q-Y,

(d) -Q-Y und

E¹ + E² zusammen E bedeuten

oder einem ihrer reaktionsfähigen Derivate umsetzt, oder daß man eine Verbindung der Formel IV

X-V-CHR²-CO-Z-(CH₂)ₐ-CO-W-E-Q-Y    IV

mit einem Reduktionsmittel behandelt,

und/oder daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine freie Amino- oder Hydroxygruppe acyliert und/oder eine Ketogruppe zu einer CHOH-Gruppe reduziert oder zu einer CH(NH₂)-Gruppe reduktiv aminiert und/oder eine Verbindung der Formel I durch Behandeln mit einer Säure in eines ihrer Salze überführt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verwendung von Verbindungen der Formel I oder von deren physiologisch unbedenklichen Salzen bei der Bekämpfung der reninabhängigen Hypertension oder des Hyperaldosteronismus.

7. Verwendung von Verbindungen der Formel I oder von deren physiologisch unbedenklichen Salzen zur Herstellung eines Arzneimittels zur Bekämpfung der reninabhängigen Hypertension oder des Hyperaldosteronismus.